# EUROPEAN PATENT APPLICATION

(11) **EP 4 446 320 A1**
(43) Date of publication of application: **16.10.2024**
(21) Application number: 22904710.5
(22) Date of filing: 09.12.2022
(51) Int. Cl.: C07D 405/14, A61K 31/506, A61P 35/00

(54) **NOVEL HETEROCYCLIC-SUBSTITUTED PYRIMIDINE DERIVATIVE EXHIBITING CANCER CELL GROWTH INHIBITORY EFFECT, AND PHARMACEUTICAL COMPOSITION CONTAINING SAME**

(30) Priority: 09.12.2021 KR 20210175542; 08.12.2022 KR 20220170783
(71) Applicant: Oncobix Co., Ltd., Yongin-si, Gyeonggi-do 16950 (KR)
(72) Inventor: JEON, Hye Min, Yongin-si, Gyeonggi-do 16950 (KR); BAEK, Min Seo, Yongin-si, Gyeonggi-do 16950 (KR); WOO, Su Jin, Yongin-si, Gyeonggi-do 16950 (KR); SHIN, Woon Seong, Yongin-si, Gyeonggi-do 16950 (KR); HA, Tae Hwan, Yongin-si, Gyeonggi-do 16950 (KR); LEE, Sun Ho, Yongin-si, Gyeonggi-do 16950 (KR); KIM, Sung Eun, Yongin-si, Gyeonggi-do 16950 (KR); KIM, Min Jung, Yongin-si, Gyeonggi-do 16950 (KR)
(74) Representative: dompatent von Kreisler Selting Werner - Partnerschaft von Patent- und Rechtsanwälten mbB
(86) International application number: PCT/KR2022/020021
(87) International publication number: WO 2023/106881

(57) **Abstract**

The present invention relates to a novel heterocyclic-substituted pyrimidine derivative represented by Chemical Formula I and a pharmaceutically acceptable salt thereof, wherein the novel heterocyclic pyrimidine derivative can effectively inhibit ALK or EGFR enzyme activity, and thus can treat carcinomas or hyperproliferative diseases in which the enzyme activity is overexpressed.

## Description

### [Technical Field]

The present application claims the benefit of priority based on Korean Patent Application No. 10-2021-0175542 filed on December 9, 2021 and Korean Patent Application No. 10-2022-0170783 filed on December 8, 2022, and the entire contents of which are incorporated as part of this specification.

The present invention relates to a novel heterocyclic-substituted pyrimidine derivative that effectively inhibits the growth of various cancer cells and a pharmaceutical composition comprising the same.

### [Background Art]

Non-Small Cell Lung Cancer (NSCLC), which occurs due to various causes, is a disease with a very high cancer-related disease prevalence and mortality rate. Non-Small Cell Lung Cancer is mainly caused by mutations and overexpression of Tyrosine kinase enzymes, and anticancer drugs developed to date are mainly aimed at inhibiting the activity of cancer-causing enzymes related to these specific mutations.

The Non-Small Cell Lung Cancer is caused by mutations in EGFR, known as the epidermal growth factor receptor, as well as expression, fusion, and rearrangement of various tumor genes, especially ALK, KRAS, and ROS1. ALK (Anaplastic Lymphoma Kinase) gene abnormality (EML4-ALK transfusion) is observed in approximately 12% of Non-Small Cell Lung Cancer patients. ALK-positive Non-Small Cell Lung Cancer is caused by the fusion of the ALK and EML4 genes. The fusion of the two genes accelerates the cell growth rate caused by the ALK gene, and is known to rapidly transform cells that receive this signal into cancer cells (tumor cell transformation).

Typically, Crizotinib, the first-line treatment for ALK-positive cancer, was approved by the U.S. FDA in 2011 as a multitargeted anticancer treatment, and is used to treat metastatic, ALK-positive Non-Small Cell Lung Cancer by inhibiting the activities of c-MET, ALK, and ROS1.

As with other anticancer drugs, the use of Crizotinib inevitably leads to the development of resistance, which is reported to be mainly due to secondary mutations in the ALK kinase domain (about 30%), ALK fusion mutation gene amplification, and activation of bypass signaling processes.

Accordingly, Crizotinib-resistant patients use ceritinib, alectinib, and brigatinib as the second-line treatment, and patients resistant to the second-line treatment use Lolartinib as the third-line treatment.

In ALK-positive Non-Small Cell Lung Cancer, which is caused by ALK fusion, mutations that impede the binding of the kinase protein to the drug are known to be the main resistance mechanism. It has been reported that these mutations affect downstream signaling within the cell, preventing the drug from working further.

Although the development of ALK fusion protein inhibitors continues to progress, the development of inhibitors that can solve the resistance problem is progressing relatively very slowly. Therefore, there is a need to develop a drug that effectively inhibits the growth of ALK mutant cancer cells, which is the main drug resistance mechanism described above.

### [Prior Art Documents]

### [Non-Patent Documents]

Nature Reviews Clinical Oncology(2018)15 694-708. Making the first move in EGFR-driven or ALK-driven NSCLC: first-generation or next - generation TKIs

PLoS ONE(2020)15(2). ALK inhibitors for non-small cell lung cancer: A systematic review and network meta-analysis.

Oncotarget(2016)7(1) 1066-75. Rearranged EML4-ALK fusion transcripts sequester in circulating blood platelets and enable blood-based crizotinib response monitoring in non-small-cell lung cancer.

### [Disclosure]

### [Technical Problem]

As a result of efforts to develop a new compound that effectively inhibits cancer or proliferative diseases with ALK mutation or EGFR mutation, the present inventors discovered a heterocyclic-substituted pyrimidine derivative containing a novel N-Alkyl alkylsulfonamide functional group that is effective in cancer treatment.

It is an object of the present invention to provide a heterocyclic-substituted pyrimidine derivative containing a novel N-Alkyl alkylsulfonamide functional group that is effective in treating cancer or proliferative diseases.

It is another object of the present invention to provide a pharmaceutical composition for the treatment of cancer or proliferative diseases with ALK overexpression, ALK mutation, or EGFR mutation, which contains the heterocyclic-substituted pyrimidine derivative containing the novel N-Alkyl alkylsulfonamide functional group.

It is still another object of the present invention to provide a pharmaceutical composition for treating Non-Small Cell Lung Cancer with ALK mutation or EGFR mutation.

### [Technical Solution]

In order to achieve the above objects, the present invention provides a compound represented by the following Chemical Formula I or a pharmaceutically acceptable salt thereof:

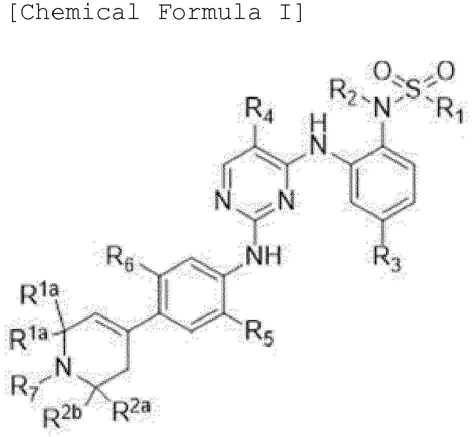

In the Chemical Formula I,
R₁ is C1 to C4 alkyl group, C3 to C6 cycloalkyl group, CF₃, or NR⁸R⁹, wherein R⁸ and R⁹ are each independently hydrogen or C1 to C4 alkyl group,
R₂ is hydrogen, C1 to C4 alkyl group, C3 to C6 cycloalkyl group, or -NR¹⁰R¹¹, wherein R¹⁰ and R¹¹ are each independently hydrogen or C1 to C4 alkyl group,
R₃ is hydrogen or halogen group,
R₄ is hydrogen, halogen, OH, CN, CF₃, C1 to C4 alkoxy, or C1 to C4 alkyl group,
R₅ is C1 to C4 alkoxy group, C3 to C6 cycloalkoxy group, CF₃substituted C1 to C4 alkoxy group, C1 to C4 alkyl group, C3 to C6 cycloalkyl group, or C3 to C6 heterocycloalkyl group, wherein the heterocycloalkyl group contains 1 to 3 heteroatoms selected from N, O and S,
R₆ is hydrogen, halogen group, C1 to C4 alkyl group, or C3 to C6 cylcoalkyl group,
R^{1a}, R^{1b}, R^{2a}, and R^{2b} are each independently hydrogen, C1 to C4 alkyl group, or halogen, and
R₇ is hydrogen, C1 to C4 alkyl group, C3 to C6 cycloalkyl group, or C3 to C8 heterocycloalkyl group, wherein the heterocycle contains 1 to 3 heteroatoms selected from N, O and S.

The compound represented by Chemical Formula I or a pharmaceutically acceptable salt thereof may be used for treating cancer or hyperproliferative disease.

In addition, the present invention
provides a pharmaceutical composition for the treatment of cancer or hyperproliferative disease containing the compound represented by Chemical Formula I or a pharmaceutically acceptable salt thereof as an active ingredient, and a pharmaceutically acceptable carrier.

The cancer or hyperproliferative disease may be caused by ALK overexpression, ALK mutation, or EGFR mutation.

In addition, the present invention provides a method of treating an animal suffering from cancer or hyperproliferative disease, comprising administering an effective amount of the compound represented by Chemical Formula I or a pharmaceutically acceptable salt thereof to the animal.

The cancer or hyperproliferative disease may be caused by ALK overexpression, ALK mutation, or EGFR mutation.

### [Advantageous Effects]

The novel heterocyclic-substituted pyrimidine derivative compound of the present invention or a pharmaceutically acceptable salt thereof effectively inhibits the enzyme activities of ALK, G120R ALK and L1196M ALK and the enzyme activities of del19/L858R/T790M EGFR and del19/T790M/C797S.

In addition, the novel heterocyclic-substituted pyrimidine derivative compound of the present invention or a pharmaceutically acceptable salt thereof effectively inhibits the growth of cancer cells with ALK overexpression, ALK mutation, or EGFR mutation. Therefore, it can be useful for treating cancer or proliferative diseases with ALK overexpression, ALK mutation, or EGFR mutation.

### [Best Mode]

Hereinafter, the present invention will be described in detail through examples. However, this is presented as an example, and the present invention is not limited thereby, and the present invention is only defined by the scope of the claims described below. In addition, even if the configuration is essential for carrying out the present invention, detailed description will be omitted for configurations that can be easily implemented using known techniques by a person skilled in the art.

Hereinafter, unless otherwise described, the term "compound of the present invention" or "compound of Chemical Formula I" is used as a concept including both the compound itself and its salts.

As used herein, the term "C1 to C4 alkyl group" refers to a straight-chain and branched hydrocarbon group having a specified number of carbon atoms. The alkyl group may be, for example, methyl, ethyl, n-propyl, i-propyl, n-butyl, s-butyl, i-butyl, t-butyl, etc.

As used herein, the term "C3 to C6 cycloalkyl group" may be selected from cyclopropyl group, cyclobutyl group, cyclopentyl group, or cyclohexyl group.

As used herein, the term "C1 to C4 alkoxy group" may be straight-chain or branched-chain. Specifically, it may be selected from methoxy, ethoxy, n-propoxy, or isopropoxy.

As used herein, the term "alkylsulfonyl" means alkyl-S(O₂)-, wherein the alkyl is defined above.

As used herein, the term "halogen" may be selected from F, Cl, Br, or I.

The present invention relates to a compound represented by the following Chemical Formula I or a pharmaceutically acceptable salt thereof.

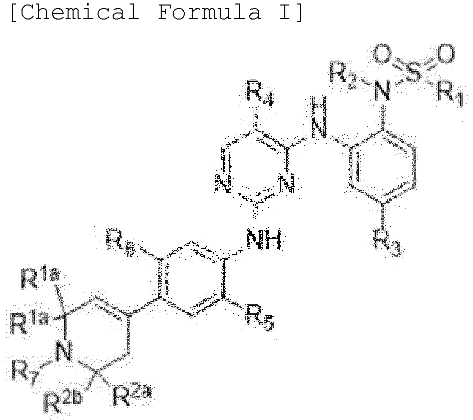

In the Chemical Formula I,
R₁ is C1 to C4 alkyl group, C3 to C6 cycloalkyl group, CF₃, or -NR⁸R⁹, wherein R⁸ and R⁹ are each independently hydrogen or C1 to C4 alkyl group,
R₂ is hydrogen, C1 to C4 alkyl group, C3 to C6 cycloalkyl group, or -NR¹⁰R¹¹, wherein R¹⁰ and R¹¹ are each independently hydrogen or C1 to C4 alkyl group,
R₃ is hydrogen or halogen group,
R₄ is hydrogen, halogen, OH, CN, CF₃, C1 to C4 alkoxy, or C1 to C4 alkyl group,
R₅ is C1 to C4 alkoxy group, C3 to C6 cycloalkoxy group, CF₃-substituted C1 to C4 alkoxy group, C1 to C4 alkyl group, C3 to C6 cylcoalkyl group, or C3 to C6 heterocycloalkly group, wherein the heterocycloalkyl group contains 1 to 3 heteroatoms selected from N, O and S,
R₆ is hydrogen, halogen group, C1 to C4 alkyl group, or C3 to C6 cycloalkyl group,
R^{1a}, R^{1b}, R^{2a}, and R^{2b} are each independently hydrogen, C1 to C4 alkyl group, or halogen, and
R₇ is hydrogen, C1 to C4 alkyl group, C3 to C6 cycloalkyl group, or C3 to C8 heterocycloalkyl group, wherein the heterocycle contains 1 to 3 heteroatoms selected from N, O and S.

In the Chemical Formula I, preferably,
R₁ is C1 to C4 alkyl group, C3 to C6 cycloalkyl group, or - NR⁸R⁹, wherein R⁸ and R⁹ are each independently hydrogen or C1 to C4 alkyl group,
R₂ is hydrogen, C1 to C4 alkyl group, or C3 to C6 cycloalkyl group,
R₃ is hydrogen or halogen group,
R₄ is hydrogen, halogen, OH, CN, or CF₃,
R₅ is C1 to C4 alkoxy group, C3 to C6 cycloalkoxy group, or CF₃-substituted C1 to C4 alkoxy group,
R₆ is hydrogen, C1 to C4 alkyl group, or C3 to C6 cycloalkyl group,
R^{1a}, R^{1b}, R^{2a}, and R^{2b} are hydrogen,
R₇ is C3 to C6 cycloalkyl group or C3 to C8 heterocycloalkyl group, wherein the heterocycle contains 1 to 3 heteroatoms selected from N, O and S.

In the Chemical Formula I, more preferably,
R₁ is C1 to C4 alkyl group, C3 to C6 cycloalkyl group, or - NH₂,
R₂ is hydrogen, C1 to C4 alkyl group, or C3 to C6 cycloalkyl group,
R₃ is hydrogen,
R₄ is halogen,
R₅ is C1 to C4 alkoxy group, C3 to C6 cycloalkoxy group, or CF₃-substituted C1 to C4 alkoxy group,
R₆ is C1 to C4 alkyl group,
R^{1a}, R^{1b}, R^{2a}, and R^{2b} are hydrogen,
R₇ is C5 to C8 heterocycloalkyl group, wherein the heterocycle may contain 1 heteroatom selected from O and S.

The Chemical Formula I compound may specifically include the following compounds.
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylmethanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)methanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylcyclopropanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylpropane-2-sulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-cyclopropylmethanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-isopropylmethanesulfonamide,
N-(2-((5-chloro-2-((5-methyl-4-(1-(tetrahydro-2H-pyran-4-yl)-1,2,3,6-tetrahydropyridin-4-yl)-2-(2,2,2-trifluoroethoxy)phenyl)amino)pyrimidin-4-yl)amino)phenyl)-N-methylmethanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylsulfonamide.

The compounds represented by Chemical Formula I of the present invention effectively inhibit the enzyme activities of ALK, G120R ALK and L1196M ALK and the enzyme activities of del19/L858R/T790M EGFR and del19/T790M/C797S. Additionally, the compounds of Chemical Formula I of the present invention have very excellent enzyme inhibitory activity compared to Crizotinib.

Therefore, the compound represented by Chemical Formula I of the present invention or a pharmaceutically acceptable salt thereof can be used as a drug that can treat or prevent cancer or hyperproliferative disease. The cancer or hyperproliferative disease may be a disease caused by ALK overexpression or ALK mutation or a disease caused by EGFR mutation.

The above cancers may be selected from the group consisting of liver cancer, stomach cancer, pancreatic cancer, colon cancer, leukemia, myeloproliferative/myelodysplastic disease, dermatofibrosarcoma, breast cancer, lung cancer, thyroid cancer, prostate cancer, ovarian cancer, brain tumor, osteosarcoma, and head and neck cancer. Additionally, the lung cancer may be small cell lung cancer or non-small cell lung cancer.

Additionally, the cancer may be Non-Small Cell Lung Cancer caused by ALK overexpression, ALK mutation, or EGFR mutation, and the cancer may be secondary cancer that has spread to other organs from the various types of cancer.

The compound represented by the Chemical Formula I according to the present invention can be used in the form of a pharmaceutically acceptable salt derived from an inorganic acid or an organic acid. The salt refers to a salt commonly used in the pharmaceutical industry to which the present invention pertains. Specifically, it may be a salt derived from one or more acids selected from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, or toluenesulfonic acid.

The compounds of the present invention may also be in the form of solvates. "Solvate" means one or more solute molecules, i.e. a compound represented by Chemical Formula I or a pharmaceutically acceptable salt thereof; and a complex, or aggregate, formed by one or more solvent molecules. The solvate may be a complex or aggregate formed with various solvent molecules such as water, methanol, ethanol, isopropanol, or acetic acid.

The compounds of the present invention may also be in the form of its stereoisomers. The stereoisomers include all stereoisomers such as enantiomers and diastereomers. The compound may be in stereoisomerically pure form or a mixture of one or more stereoisomers, for example a racemic mixture. Separation of specific stereoisomers can be performed by one of the conventional methods known in the art. Some examples of the compounds of the present invention may have greater anticancer effects of certain stereoisomers than their racemic mixtures. In this case, the dosage can be reduced by using a specific stereoisomer. Therefore, cancer can be treated efficiently by separating specific stereoisomers, for example, enantiomers or partial structural isomers, which have a high killing effect on cancer cells.

According to another aspect of the present invention, the present invention provides a pharmaceutical composition for treating or preventing cancer or hyperproliferative disease, comprising the compound of the present invention or a pharmaceutically acceptable salt thereof as the active ingredient, and a pharmaceutically acceptable carrier. The cancer or hyperproliferative disease may be a disease caused by ALK overexpression or ALK mutation or a disease caused by EGFR mutation.

Since the pharmaceutical composition of the present invention uses the above-described Chemical Formula I or its salt as an active ingredient, details in common with the above-described content are omitted to avoid excessive duplication of the specification.

The pharmaceutical composition of the present invention can be formulated according to conventional methods. It can be prepared in various oral administration forms such as tablets, pills, powders, capsules, syrups, emulsions, and microemulsions, or prepared for parenteral administration forms such as intravenous injection, subcutaneous injection, intramuscular injection, intraperitoneal injection, transdermal injection, or direct injection into the tissue.

As used herein, "pharmaceutically acceptable carrier" refers to a substance, generally an inert substance, used in combination with an active ingredient to aid application of the active ingredient. The carrier includes, for example, one or more selected from fillers, binders, disintegrants, buffers, preservatives, antioxidants, lubricants, flavoring agents, thickeners, coloring agents, emulsifiers, suspending agents, stabilizers, pH adjusters, and isotonic agents.

Examples of carriers used in the present invention include cellulose, calcium silicate, lactose, dextrose, corn starch, sucrose, calcium phosphate, stearic acid, magnesium stearate, calcium stearate, gelatin, talc, surfactant, emulsifiers, suspending agents, diluents, etc. When the pharmaceutical composition of the present invention is prepared in the form of an injection, the carriers may be water, saline solution, aqueous glucose solution, aqueous pseudosaccharide solution, alcohol, glycol, oil, fatty acid, fatty acid ester, glyceride, surfactant, suspending agent, emulsifier, etc.

According to another aspect of the present invention, the present invention provides a method of preventing or treating a disease by administering the compound of the present invention or a pharmaceutically acceptable salt thereof to an individual in need of prevention or treatment of the aforementioned disease.

The dosage of the pharmaceutical composition of the present invention may vary depending on various factors such as the patient's condition, route of administration, and the judgment of the attending physician. Effective dosages can be estimated from dose-response curves obtained from in vitro experiments or animal model tests. The proportion and concentration of the compounds of the present invention present in the administered composition may be determined depending on the chemical nature, route of administration, therapeutic dosage, etc.

For example, the dosage of the pharmaceutical composition of the present invention may be 0.0001 mg/kg (body weight) to 100 mg/kg (body weight) based on the compound of Chemical Formula I.

The pharmaceutical composition of the present invention may additionally include one or more selected from immunomodulators, chemical anticancer drugs, and immuno-anticancer drugs.

Cytokines suitable for use as the immunomodulator include interleukin (e.g., IL-1, IL-2, IL-4, IL-5, IL-6, IL-7, IL-12, etc.), macrophage colony stimulating factor (M-CSF), tumor necrosis factor (TNF), or granulocyte, macrophage colony stimulating factor (GM- CSF), etc., but is not limited thereto.

The chemical anticancer agents include, but are not limited to, Gemcitabine, Cytarabine, Carboplatin, Cisplatin, Crizotinib, Cyclophosphamide, Docetaxel, Doxorubicin, Etoposide, 5-fluorouracil, irinotecan, Methotrexate, Paclitaxel, Topotecan, Trabectedin, Vincristine, or Vinbrastine.

The immuno-anticancer drug may be an immune checkpoint inhibitor for any one selected from the group consisting of CTLA-4(cytotoxic T-lymphocyte-associated protein 4), PD-1(Programmed cell death protein 1), LAG-3(Lymphocyte Activation Gene-3), TIM-3(T-cell Immunoglobulin and Mucin-domain containing-3), TIGIT(T-cell Immunoreptor with IG and ITIM domain), or VISTA(V-domain Ig Suppressor of T cell Activation), but is not limited thereto.

Hereinafter, the present invention will be described in more detail through examples. These examples are intended to illustrate the present invention in more detail, and it is obvious to those skilled in the art that the scope of the present invention is not limited by these examples according to the gist of the present invention.

### Example

The compound of Chemical Formula I according to the present invention can be prepared, for example, by the method of Reaction Scheme I below.:

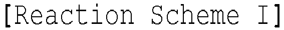

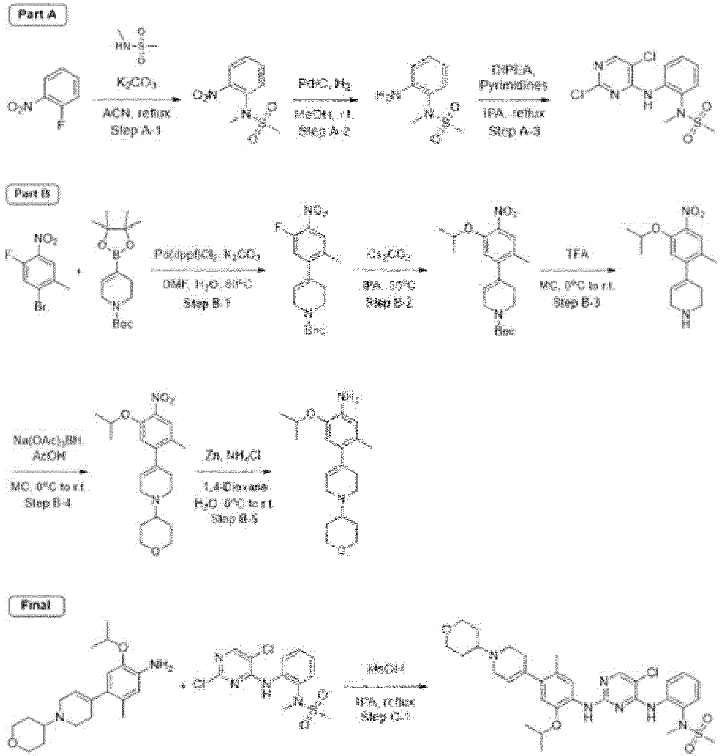

### Step A-1: Synthesis of N-methyl-N-(2-nitrophenyl)methanesulfonamide

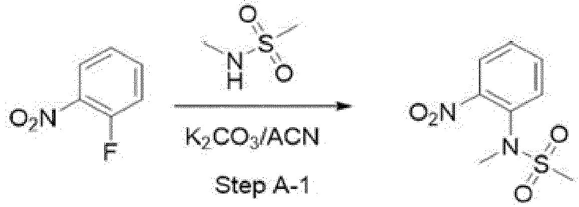

1-Fluoro-2-nitrobenzene(1.0 equivalent) is dissolved in acetonitrile, and potassium carbonate(2.0 equivalent) and N-methylmethanesulfonamide(1.4 equivalent) are added thereto at room temperature followed by stirring at 80°C overnight. After completion of the reaction, the temperature is lowered to room temperature and filtered. The filtrate is evaporated under reduced pressure to obtain the compound and used in the next reaction without separation.

### Step A-2: Synthesis of N-(2-aminophenyl)-N-methylmethanesulfonamide

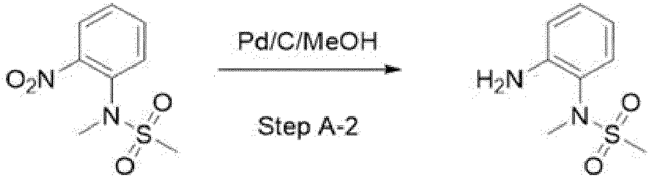

N-methyl-N-(2-nitrophenyl)methanesulfonamide(1.0 equivalent) is dissolved in methanol, ethyl acetate(1:1) and 10% Palladium/Charcoal(0.2 equivalent) is added thereto followed by stirring for 2 hours under hydrogen. After completion of the reaction, it is filtered using Celite. The filtrate was evaporated under reduced pressure and then solidified using ethylether and pentane. This is filtered to obtain the target compound and used in the next reaction without separation.

### Step A-3: Synthesis of N-(2-((2,5-dichloropyrimidin-4-yl)amino)phenyl)-N-methylmethanesulfonamide

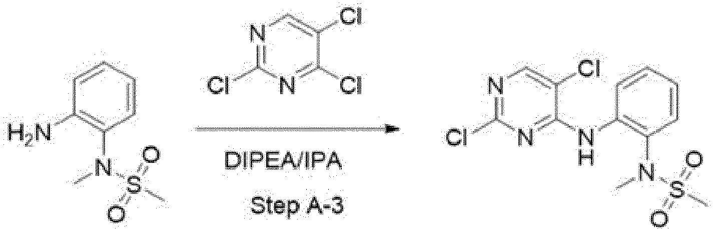

N-(2-aminophenyl)-N-methylmethanesulfonamide(1.0 equivalent) is dissolved in isopropyl alcohol, 2,4,5-trichloropyridine(1.1 equivalent) and N,N-diisopropylethylamine(2.5 equivalent) are added thereto at room temperature followed by stirring at 80°C overnight. After completion of the reaction, the filtrate is evaporated under reduced pressure and extracted using water and dichloromethane. The organic layer is washed using 2N hydrochloric acid. The organic layer is evaporated under reduced pressure to obtain the target compound and used in the next reaction without separation.

### Step B-1: Synthesis of tert-butyl 4-(5-fluoro-2-methyl-4-nitrophenyl)-3,6-dihydropyridine-1(2H)-carboxylate

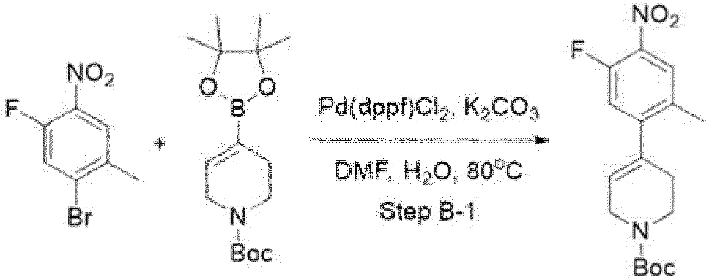

1-Bromo-5-fluoro-2-methyl-4-nitrobenzene(1.0 equivalent) is dissolved in 1,2-dimethoxymethane and water(10:1). Tert-butyl 4-(4,4,5,5-tetramethyl-1,3,2-dioxaboran-2-yl)-3,6-dihydropyridine-1(2H)-carboxylate(1.0 equivalent), potassium carbonate(3.0 equivalent) and (1,1'-bis(diphenytlphospino)ferrocene)palladium(II) dichloride(0.04 equivalent) are added thereto at room temperature and then reluxed under nitrogen. After completion of the reaction, extraction is performed with water and ethyl acetate. The organic layer is collected, evaporated under reduced pressure, and the target compound is obtained using column chromatography (hexane:ethyl acetate = 3:1).

### Step B-2: Synthesis of tert-butyl 4-(5-isopropoxy-2-methyl-4-nitrophenyl)-3,6-dihydropyridine-1(2H)-carboxylate

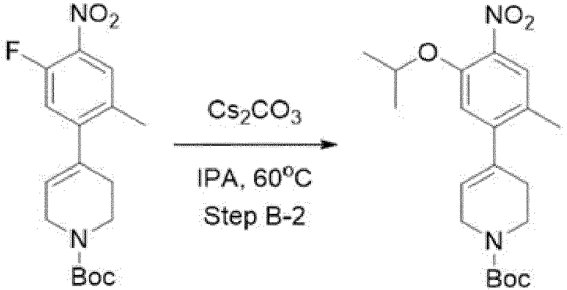

Tert-butyl 4-(5-fluoro-2-methyl-4-nitrophenyl)-3,6-dihydropyridine-1(2H)-carboxylate(1.0 equivalent) is dissolved in isopropyl alcohol and cesium carbonate(3.0 equivalent) is added thereto at room temperature followed by stirring at 60°C overnight. After completion of the reaction, extraction is performed with water and ethyl acetate. The organic layer is collected, evaporated under reduced pressure, and the target compound is obtained using column chromatography (hexane:ethyl acetate = 4:1).

### Step B-3: Synthesis of 4-(5-isopropoxy-2-methyl-4-nitrophenyl)-1,2,3,6-tetrahydropyridine

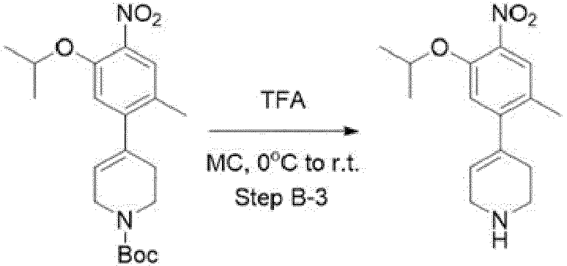

Tert-butyl 4-(5-isopropoxy-2-methyl-4-nitrophenyl)-3,6-dihydropyridine-1(2H)-carboxylate(1.0 equivalent) is dissolved in dichloromethane and trifluoro acetic acid(1.0 equivalent) is added thereto at 0°C. After completion of the reaction, it is neutralized with 1N sodium hydroxide and extracted with water and dichloromethane. The organic layer is collected, evaporated under reduced pressure, and used in the next reaction without separation.

### Step B-4: Synthesis of 4-(5-isopropoxy-2-methyl-4-nitrophenyl)-1-(tetrahydro-2H-pyran-4-yl)-1,2,3,6-tetrahydropyridine

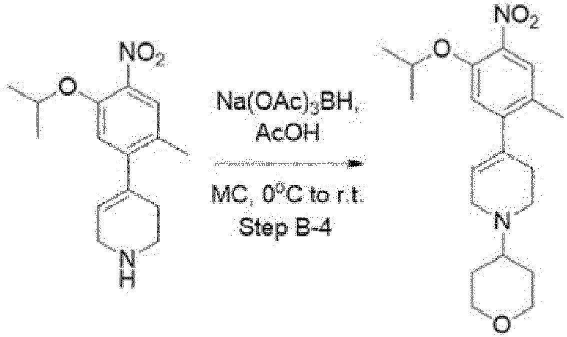

4-(5-Isopropoxy-2-methyl-4-nitrophenyl)-1,2,3,6-tetrahydropyridine(1.0 equivalent) is dissolved in toluene and tetrahydro-4H-pyran-4-one(1.2 equivalent), triethyl amine(2.58 equivalent), acetic acid(1.53 equivalent) and sodium triacetoxyborohydride(0.83 equivalent, 3 times) are added thereto at room temperature followed by stirring at room temperature overnight. After completion of the reaction, extraction is performed with water and ethyl acetate. The organic layer is collected, evaporated under reduced pressure, and used in the next reaction without separation.

### Example 1: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylmethanesulfonamide

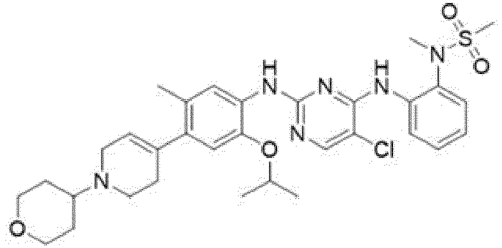

### Step B-5: Synthesis of 2-isopropoxy-5-methyl-4-(1-(tetrahydro-2H-pyran-4-yl)-1,2,3,6-tetrahydropyridin-4-yl)aniline

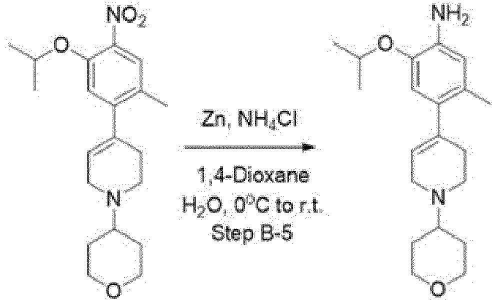

4-(5-Isopropoxy-2-methyl-4-nitrophenyl)-1-(tetrahydro-2H-pyran-4-yl)-1,2,3,6-tetrahydropyridine(1.0 equivalent) is dissolved in 1,4-dioxane and water(3:1) and zinc(10.0 equivalent) and ammonium chloride(10.0 equivalent) are added thereto at 0°C followed by stirring at room temperature overnight. After completion of the reaction, extraction is performed with water and ethyl acetate. The organic layer is collected, evaporated under reduced pressure, and used in the next reaction without separation.

### Step C-1: Synthesis of final compound

Pyrimidine derivative(1.0 equivalent) is dissolved in isopropyl alcohol and aniline derivative(1.0 equivalent) and methanesulfonic acid(1.3 equivalent) are added thereto at room temperature followed by stirring at 80°C overnight. After completion of the reaction, the solvent is removed by evaporation under reduced pressure and extracted using a mixture of water and dichloromethane. The organic layer is evaporated under reduced pressure, and the target compound is obtained using column chromatography (methyl alcohol:dichloromethane=10:1).

Yield: 15.6%; Pale yellow solid; 1H NMR(400 MHz, DMSO-d6) δ 8.37(s, 1H), 8.18 - 8.12(m, 2H), 7.83(s, 1H), 7.64 - 7.55(m, 2H), 7.30(td, J = 8.4, 7.9, 1.5 Hz, 1H), 7.20(td, J = 7.6, 1.6 Hz, 1H), 6.67(s, 1H), 5.51 - 5.46(m, 1H), 4.53(hept, J = 6.1 Hz, 1H), 3.87(dd, J = 10.9, 4.2 Hz, 2H), 3.26 - 3.23(m, 3H), 3.18 - 3.10(m, 5H), 3.05(s, 3H), 2.65(t, J = 5.5 Hz, 2H), 2.24(m, 2H), 2.03(s, 3H), 1.72(m, 2H), 1.42 (qd, J = 12.2, 4.4 Hz, 2H), 1.29 - 1.12(m, 8H). MS: ESI m/z 641.2664 [M+H]+

### Example 2: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)methanesulfonamide

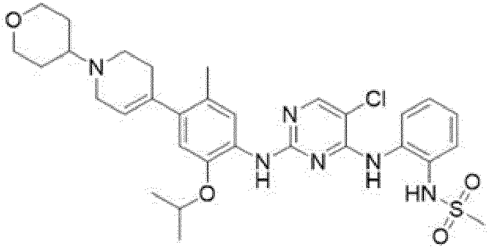

Synthesis was performed using the method of Step C-1.
Yield: 15.5%; Yellow solid; 1H NMR(500 MHz, DMSO-D6) δ 9.33(s, 1H), 8.64(s, 1H), 8.16(s, 1H), 7.88(d; J = 7.8 Hz, 1H), 7.69(s, 1H), 7.63(s, 1H), 7.41(dd; J = 7.4, 2.0 Hz, 1H), 7.29 - 7.19(m, 2H), 6.68 (s, 1H), 5.50(d; J = 4.0 Hz, 1H), 4.56(p, J = 6.1 Hz, 1H), 3.95 - 3.87(m, 2H), 3.31 - 3.27(m, 2H), 3.17(d; J = 3.4 Hz, 2H), 2.92(s, 3H), 2.70(t, J = 5.5 Hz, 2H), 2.26 (s, 2H), 1.98(s, 3H), 1.77(d; J = 13.0 Hz, 2H), 1.48(tt, J = 12.0, 6.1 Hz, 2H), 1.24(d; J = 6.0 Hz, 6H). MS: ESI m/z 626.2433 [M]+

### Example 3: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylcyclopropanesulfonamide

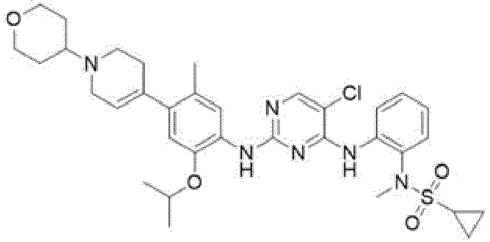

Synthesis was performed using the method of Step C-1.
Yield: 27.2%; Yellow solid; ¹H NMR(500 MHz, DMSO-D6) δ 8.38(s, 1H), 8.19(s, 1H), 8.17(s, 1H), 7.87(s, 1H), 7.70 - 7.67(m, 1H), 7.66(d, *J* = 1.8 Hz, 1H), 7.34 (t, *J* = 7.6 Hz, 1H), 7.23(td, *J* = 7.7, 1.6 Hz, 1H), 6.72(s, 1H), 5.76(s, 1H), 5.53(d, *J* = 4.3 Hz, 1H), 4.56(hept, *J* = 6.1 Hz, 1H), 3.94 - 3.88(m, 2H), 3.32 - 3.27(m, 2H), 3.22(s, 3H), 3.17(q, *J* = 3.0 Hz, 2H), 2.84(td, *J =* 7.9, 4.0 Hz, 1H), 2.70(t, *J* = 5.5 Hz, 2H), 2.29(s, 2H), 2.08(d, *J* = 2.5 Hz, 3H), 1.80 - 1.73(m, 2H), 1.47(qd, *J* = 12.2, 4.3 Hz, 2H), 1.24(d, *J* = 6.0 Hz, 6H), 1.07 - 1.02(m, 2H), 0.87(s, 2H). MS: ESI m/z 666.2 [M] +

### Example 4: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylpropane-2-sulfonamide

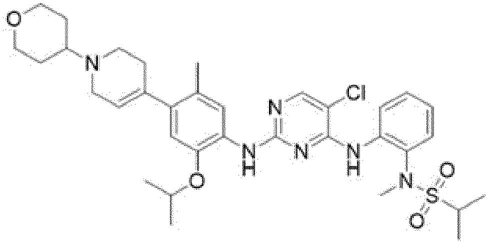

Synthesis was performed using the method of Step C-1.
Yield: 32.1%; Yellow solid; ¹H NMR(500 MHz, DMSO-D6) δ 8.44(s, 1H), 8.18(s, 1H), 7.99(d, *J* = 8.1 Hz, 1H), 7.77(s, 1H), 7.63(s, 1H), 7.60(dd, *J =* 8.0, 1.5 Hz, 1H), 7.41 - 7.34(m, 1H), 7.29(t, *J* = 7.4 Hz, 1H), 6.69(s, 1H), 5.53 - 5.48(m, 1H), 4.56(hept, *J* = 6.1 Hz, 1H), 3.94 - 3.83(m, 2H), 3.53(p, *J* = 6.7 Hz, 1H), 3.29(dd, *J* = 11.8, 2.0 Hz, 2H), 3.19(s, 3H), 3.15(q, *J* = 3.0 Hz, 2H), 2.68(t, *J* = 5.5 Hz, 2H), 2.29 - 2.23(m, 2H), 2.01(s, 3H), 1.79 - 1.72(m, 2H), 1.47(qd, *J* = 12.3, 4.5 Hz, 2H), 1.28(d, *J* = 6.7 Hz, 6H), 1.24(d, *J* = 6.0 Hz, 6H). MS: ESI m/z 668.2 [M]+

### Example 5: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-cyclopropylmethanesulfonamide

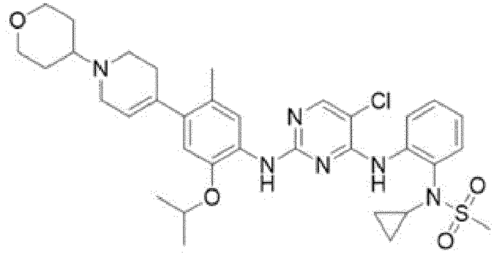

Synthesis was performed using the method of Step C-1.
Yield: 55.9%; Yellow solid; ¹H NMR(500 MHz, DMSO-D6) δ 8.22(d, *J* = 8.1 Hz, 1H), 8.19(s, 1H), 8.07(s, 1H), 7.93(s, 1H), 7.67(dd, *J* = 7.9, 1.6 Hz, 1H), 7.64(s, 1H), 7.32 (t, *J* = 7.6 Hz, 1H), 7.24(td, *J* = 7.6, 1.6 Hz, 1H), 6.72(s, 1H), 5.54(dt, *J* = 3.6, 1.9 Hz, 1H), 4.56(hept, *J* = 6.4 Hz, 1H), 3.95 - 3.88(m, 2H), 3.32 - 3.27(m, 2H), 3.24(dq, *J* = 5.5, 3.3, 2.7 Hz, 1H), 3.21(s, 3H), 3.17(q, *J* = 3.0 Hz, 2H), 2.70(t, *J* = 5.4 Hz, 2H), 2.29(d, *J* = 5.7 Hz, 2H), 2.09(d, *J* = 5.9 Hz, 3H), 1.80 - 1.73(m, 2H), 1.48(qd, *J* = 12.1, 4.4 Hz, 2H), 1.23(dd, *J* = 6.0, 4.3 Hz, 6H), 0.94 (td, *J* = 8.1, 7.5, 4.4 Hz, 2H), 0.56 - 0.47(m, 1H), 0.15(dp, *J* = 8.7, 4.6 Hz, 1H). MS: ESI m/z 666.2 [M]+

### Example 6: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-isopropylmethanesulfonamide

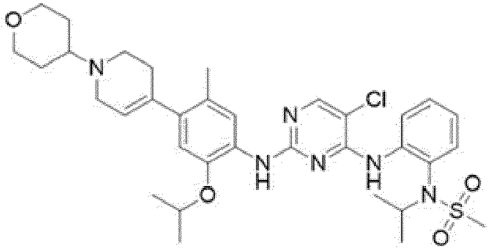

Synthesis was performed using the method of Step C-1.
Yield: 56.1%; Yellow solid; ¹H NMR(500 MHz, DMSO-D6) δ 8.21(d, *J* = 3.6 Hz, 2H), 8.16(s, 1H), 7.90(s, 1H), 7.69(s, 1H), 7.49(dd, *J* = 7.9, 1.6 Hz, 1H), 7.45 - 7.38 (m, 1H), 7.25 (td, *J* = 7.7, 1.5 Hz, 1H), 6.72(s, 1H), 5.53 (d, *J* = 3.8 Hz, 1H), 4.56(hept, *J* = 6.5, 6.0 Hz, 1H), 4.43(p, *J* = 6.7 Hz, 1H), 3.91(dd, *J* = 10.4, 4.1 Hz, 2H), 3.33 - 3.27(m, 3H), 3.17(d, *J* = 3.2 Hz, 2H), 3.15(s, 3H), 2.70(t, *J* = 5.5 Hz, 2H), 2.29 (s, 2H), 2.09(s, 2H), 2.05(s, 3H), 1.77(d, *J* = 12.2 Hz, 2H), 1.47(qd, *J* = 12.1, 4.4 Hz, 2H), 1.24(t, *J* = 6.3 Hz, 6H), 1.18(d, *J* = 6.6 Hz, 3H), 0.90(d, *J* = 6.7 Hz, 3H). MS: ESI m/z 668.2 [M]+

### Example 7: Preparation of N-(2-((5-chloro-2-((5-methyl-4-(1-(tetrahydro-2H-pyran-4-yl)-1,2,3,6-tetrahydropyridin-4-yl)-2-(2,2,2-trifluoroethoxy)phenyl)amino)pyrimidin-4-yl)amino)phenyl)-N-methylmethanesulfonamide

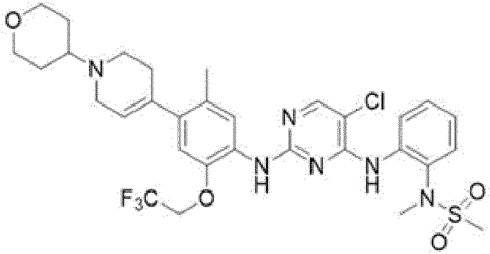

Synthesis was performed using the method of Step C-1.
Yield: 35.7%; Yellow solid; ¹H NMR(500 MHz, DMSO-D6) δ 8.38(s, 1H), 8.17(s, 1H), 8.15(d, *J* = 4.5 Hz, 2H), 7.61(dd, *J* = 7.9, 1.6 Hz, 1H), 7.51 (s, 1H), 7.27 (td, *J* = 7.8, 1.6 Hz, 1H), 7.21(td, *J* = 7.6, 1.7 Hz, 1H), 6.91(s, 1H), 5.57(dt, *J* = 3.6, 1.8 Hz, 1H), 4.69(q, *J* = 8.9 Hz, 2H), 3.95 - 3.88(m, 2H), 3.30(dd, *J* = 11.7, 2.0 Hz, 2H), 3.18(d, *J* = 2.6 Hz, 5H), 3.09(s, 3H), 2.71(t, *J* = 5.5 Hz, 2H), 2.31(dq, *J* = 5.9, 3.0 Hz, 2H), 2.12(s, 3H), 1.81 - 1.74 (m, 2H), 1.48(qd, *J* = 12.1, 4.4 Hz, 2H), 1.32 - 1.21(m, 1H). MS: ESI m/z 680.2 [M]+

### Example 8: Preparation of N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylsulfonamide

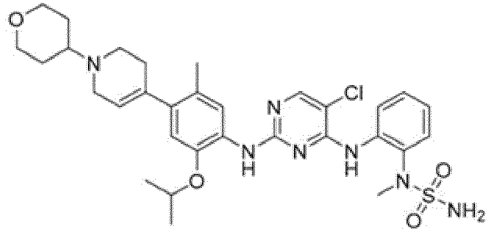

Synthesis was performed using the method of Step C-1.
Yield: 45.2%; Yellow solid; ¹H NMR (500 MHz, DMSO-D6)1H NMR (500 MHz, Chloroform-d) δ 9.21 (s, 1H), 8.52 (s, 1H), 8.43 (s, 1H), 7.43 (dd, J = 7.5, 1.6 Hz, 1H), 7.39 (dd, J = 7.4, 1.5 Hz, 1H), 7.14 (s, 1H), 7.03 (td, J = 7.4, 1.5 Hz, 1H), 6.93 (s, 2H), 6.87 (s, 1H), 6.80 (td, J = 7.5, 1.5 Hz, 1H), 6.06 (tt, J = 6.2, 1.0 Hz, 1H), 4.63 (dt, J = 13.5, 6.6 Hz, 1H), 3.70 (dt, J = 12.4, 7.1 Hz, 2H), 3.60 (dt, J = 12.6, 7.1 Hz, 2H), 3.43 (s, 3H), 3.30 (dt, J = 6.2, 1.0 Hz, 2H), 2.94 (p, J = 6.9 Hz, 1H), 2.84 (td, J = 7.1, 0.9 Hz, 2H), 2.71 (tt, J = 6.9, 0.9 Hz, 2H), 2.41 (s, 3H), 1.90 - 1.71 (m, 4H), 1.33 (d, J = 6.9 Hz, 6H). MS: ESI m/z 641.3 [M]+

### Test Example 1: Measurement of Kinase Inhibitory Activity

For the compounds of Examples 1 to 8 above, the inhibitory activity against ALK, ALK mutation (ALK(G1202R)), and EGFR mutation (ALK(L1196M)) was measured, and the results are shown in Table 1 below. Kinase inhibitory activity was measured by calculating the IC₅₀ value at the concentration at which each compound inhibited the kinase inhibitory activity. Crizotinib (APG-2449) was used as a control drug.
1. Each kinase was incubated under 8mM MOPS, pH 7.0, 0.2mM EDTA, 250µM KKKGQEEEYVFIE, 1mM sodium orthovanadate, 5mM sodium-6-glycerophosphate, 10mM Magnesium acetate, [³³P]-ATP.
2. The reaction proceeded by adding the evaluation compound (DMSO solution) and Mg/ATP.
3. After about 40 minutes at room temperature, the reaction was terminated by adding 10uL of 0.5% phosphoric acid.
4. The reaction solution was divided into 0.5% 10uL and spotted on a P30 filtermat.
5. It was washed four times with 0.425% phosphoric acid for about 4 minutes. After washing once with methanol, it was dried and analyzed by scintillation counting to measure the IC₅₀ value.

**[Table 1]**

| **Example** | **ALK** | **ALK(G1202R)** | **ALK(L1196M)** |
|---|---|---|---|
| | IC₅₀ | IC₅₀ | IC₅₀ |
| 1 | 1.1nM | 17.8nM | 0.2nM |
| 2 | 1.8nM | 25.8nM | 0.6nM |
| 3 | 0.6nM | 0.7nM | 0.05nM |
| 4 | 0.9nM | 161nM | 0.3nM |
| 5 | 3.1nM | 153nM | 0.5nM |
| 6 | 0.6nM | 254nM | 6.7nM |
| 7 | 0.5nM | 10.5nM | 0.05nM |
| 8 | 0.6nM | 17.8nM | 0.07nM |
| APG-2449 | 0.8nM | >1,000nM | 0.9nM |

As shown in the experimental results in Table 1 above, the compounds prepared by Examples of the present invention showed similar inhibitory activity on wild-type ALK kinase compared to APG-2449, but showed very excellent activity on ALK point mutant kinase.

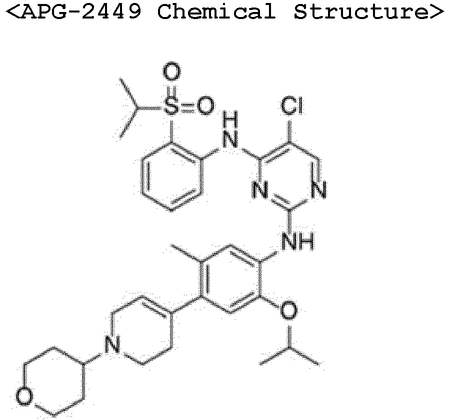

### Test Example 2: Measurement of cancer cell growth inhibitory effect

The growth inhibitory effects of the compounds in Examples 3, 7, and 8 above on ALK mutant cancer cell lines were measured. Anticancer efficacy activity was measured using H3122 (EML4-ALK v1) and H2228 (EML4-ALK v3) as ALK mutant cell lines by the following method.

Gene construction: Wild type and mutant EGFR were purchased from Addgene. All construction was done as a retroviral vector, ultimately completing the viral particle for infection.

### <Confirmation of changes in cellular kinase activity (Western blotting)>

Each cell line was treated with the compounds of Examples 3, 7, and 8 in a concentration-dependent manner, respectively, and cells were obtained after 5 hours. Cell lysates were prepared using EBC lysis buffer(50 mM Tris-HCl [pH 8.0], 120 mM NaCl, 1% Triton X-100, 1 mM EDTA, 1 mM EGTA, 0.3 mM phenylmethylsulfonylfluoride, 0.2 mM sodium orthovanadate, 0.5% NP-40, and 5 U/mL aprotinin).

### <Verification of anticancer effect through MTT assay >

2 × 10⁵ cells were seeded in a 96-well plate. 24 hours later, each of the compounds in Examples 3, 7, and 8 was treated in a dose-dependent manner, incubated for 72 hours, reacted with 15uL MTT reagent for 4 hours, and then 100uL 10% SDS was added thereto and incubated for 24 hours. The change in final OD was read at 595 nm. To analyze the MTT results, IC₅₀ values were measured using prism software.

The concentration at which each compound inhibited cell growth by 50% was calculated as the IC₅₀ value, and the results are shown in Table 2 below. APG-2449 was used as a control drug.

**[Table 2]**

| Example | H3122 (IC₅₀) | H2228 (IC₅₀) |
|---|---|---|
| 3 | 31.9nM | 43.1nM |
| 7 | 39.1nM | 16.8nM |
| 8 | 22.1nM | 20.8nM |
| APG-2449 | 87.8nM | 62.9nM |

As shown in the experimental results in Table 2 above, the compounds prepared by Examples of the present invention were confirmed to exhibit significant cancer cell growth inhibitory activity compared to APG-2449 against cancer cell lines expressing mutations.

## Claims

1. A compound represented by the following Chemical Formula I or a pharmaceutically acceptable salt thereof; 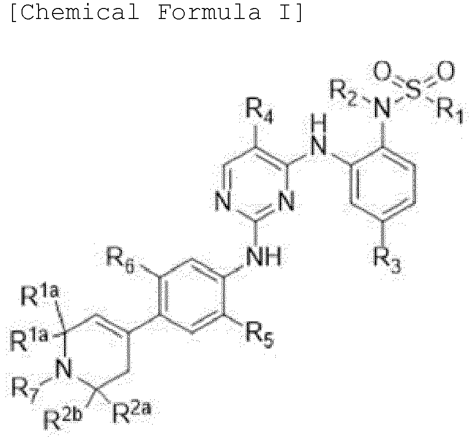 in the Chemical Formula I,
R₁ is C1 to C4 alkyl group, C3 to C6 cycloalkyl group, CF₃, or -NR⁸R⁹, wherein R⁸ and R⁹ are each independently hydrogen or C1 to C4 alkyl group,
R₂ is hydrogen, C1 to C4 alkyl group, C3 to C6 cycloalkyl group, or -NR¹⁰R¹¹, wherein R¹⁰ and R¹¹ are each independently hydrogen or C1 to C4 alkyl group,
R₃ is hydrogen or halogen group,
R₄ is hydrogen, halogen, OH, CN, CF₃, C1 to C4 alkoxy, or C1 to C4 alkyl group,
R₅ is C1 to C4 alkoxy group, C3 to C6 cycloalkoxy group, CF₃-substituted C1 to C4 alkoxy group, C1 to C4 alkyl group, C3 to C6 cycloalkyl group, or C3 to C6 heterocycloalkyl group, wherein the heterocycloalkyl group contains 1 to 3 heteroatoms selected from N, O and S,
R₆ is hydrogen, halogen group, C1 to C4 alkyl group, or C3 to C6 cycloalkyl group,
R^{1a}, R^{1b}, R^{2a}, and R^{2b} are each independently hydrogen, C1 to C4 alkyl group, or halogen, and
R₇ is hydrogen, C1 to C4 alkyl group, C3 to C6 cycloalkyl group, or C3 to C8 heterocycloalkyl group, wherein the heterocycle contains 1 to 3 heteroatoms selected from N, O and S.

2. The compound represented by Chemical Formula I or a pharmaceutically acceptable salt thereof according to claim 1, wherein R₁ is C1 to C4 alkyl group, C3 to C6 cylcoalkyl group, or -NR⁸R⁹, wherein R⁸ and R⁹ are each independently hydrogen or C1 to C4 alkyl group,
R₂ is hydrogen, C1 to C4 alkyl group, or C3 to C6 cycloalkyl group,
R₃ is hydrogen, or halogen group,
R₄ is hydrogen, halogen, OH, CN, or CF₃,
R₅ is C1 to C4 alkoxy group, C3 to C6 cycloalkoxy group, or CF₃-substituted C1 to C4 alkoxy group,
R₆ is hydrogen, C1 to C4 alkyl group, or C3 to C6 cycloalkyl group,
R^{1a}, R^{1b}, R^{2a}, and R^{2b} are hydrogen, and
R₇ is C3 to C6 cycloalkyl group or C3 to C8 heterocycloalkyl group, wherein the heterocycle contains 1 to 3 heteroatoms selected from N, O and S.

3. The compound represented by Chemical Formula I or a pharmaceutically acceptable salt thereof according to claim 1, wherein the compound of Chemical Formula I is selected from the group consisting of the following compounds:
N-(2-((5-chloro-2-((2-isopropoxy-5-methyl-4-(1-(tetrahydro-2H-pyran-4-yl)-1,2,3,6-tetrahydropyridin-4-yl)phenyl) amino)pyrimidin-4-yl) amino)phenyl)-N-methylmethanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)methanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl) amino)pyrimidin-4-yl) amino)phenyl)-N-methylcyclopropanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylpropane-2-sulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-cyclopropylmethanesulfonamide,
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-isopropylmethanesulfonamide,
N-(2-((5-chloro-2-((5-methyl-4-(1-(tetrahydro-2H-pyran-4-yl)-1,2,3,6-tetrahydropyridin-4-yl)-2-(2,2,2-trifluoroethoxy)phenyl)amino)pyrimidin-4-yl)amino)phenyl)-N-methylmethanesulfonamide, and
N-(2-((5-chloro-2-((2-isopropoxy-5-methyl-4-(1-(tetrahydro-2H-pyran-4-yl)-1,2,3,6-tetrahydropyridin-4-yl)phenyl)amino)pyrimidin-4-yl)amino)phenyl)-N-methylsulfonamide.

4. A pharmaceutical composition for the treatment of cancer or hyperproliferative disease containing the compound represented by Chemical Formula I of claim 1 or a pharmaceutically acceptable salt thereof as an active ingredient, and a pharmaceutically acceptable carrier.

5. The pharmaceutical composition for the treatment of cancer or hyperproliferative disease according to claim 4, wherein the cancer or hyperproliferative disease is caused by ALK overexpression, ALK mutation, or EGFR mutation.

6. The pharmaceutical composition for the treatment of cancer or hyperproliferative disease according to claim 5, wherein the cancer is liver cancer, stomach cancer, pancreatic cancer, colon cancer, leukemia, myeloproliferative/myelodysplastic disease, dermatofibrosarcoma, breast cancer, lung cancer, thyroid cancer, prostate cancer, ovarian cancer, brain tumor, osteosarcoma, and head and neck cancer.

7. The pharmaceutical composition for the treatment of cancer or hyperproliferative disease according to claim 4, wherein the cancer is Non-Small Cell Lung Cancer with ALK overexpression or ALK mutation, or Non-Small Cell Lung Cancer with EGFR mutation.

8. The pharmaceutical composition for the treatment of cancer or hyperproliferative disease according to claim 4, wherein the pharmaceutical composition further contains at least one selected from immunomodulator, chemical anticancer drug, and immunoanticancer drug.
